# EUROPEAN PATENT APPLICATION

(11) **EP 4 417 609 A1**
(43) Date of publication of application: **21.08.2024**
(21) Application number: 22880404.3
(22) Date of filing: 14.10.2022
(51) Int. Cl.: C07D 491/044, A61K 31/4353, A61P 9/10, A61P 9/12, A61P 9/04, A61P 9/00, A61P 15/10, A61P 3/00, A61P 35/00, A61P 29/00, A61P 1/16, A61P 3/10, A61P 7/02, A61P 7/06, A61P 31/20, A61P 1/00

(54) **NEW CRYSTAL FORM AND USE OF NITROGENOUS TRICYCLIC COMPOUND**

(30) Priority: 15.10.2021 CN 202111202309
(71) Applicant: Sunshine Lake Pharma Co., Ltd., Dongguan, Guangdong 523000 (CN)
(72) Inventor: YANG, Xinye, Dongguan, Guangdong 523871 (CN); SHAN, Yuefeng, Dongguan, Guangdong 523871 (CN); CHEN, Liang, Dongguan, Guangdong 523871 (CN); WANG, Xiaojun, Dongguan, Guangdong 523871 (CN)
(74) Representative: Hoffmann Eitle
(86) International application number: PCT/CN2022/125303
(87) International publication number: WO 2023/061468

(57) **Abstract**

Provided are a new crystal form and use of a nitrogenous tricyclic compound in the field of drugs. Specifically, the present invention relates to a new crystal form of 2-((5-cyclopropyl-3-(2,6-dichlorophenyl)isoxazol-4-yl)methoxy)-10/7-spiro[benzo[6,7]oxepino[3,2-b]pyridine-11,1'-cyclopropane]-7-carboxylic acid, and the new crystal form is a crystal form I. The present invention further relates to a pharmaceutical composition comprising the crystal form I, and the use of the crystal form I or the pharmaceutical composition in preparation of a drug for preventing, treating, or relieving an FXR-mediated disease of a patient.

## Description

### TECHNICAL FIELD

The present invention belongs to the field of medicine, and relates to a new crystal form and use of a nitrogenous tricyclic compound, particularly relates to a new crystal form of 2-((5-cyclopropyl-3-(2,6-dichlorophenyl)isoxazol-4-yl)methoxy)-lOH spiro[benzo[6,7]oxepino[3,2 -b]pyridine-11,1'-cyclopropane]-7-carboxylic acid and a pharmaceutical composition thereof, and further relates to a use of the new crystal form or the pharmaceutical composition in the manufacture of a medicament, specifically, the use of the new crystal form or the pharmaceutical composition in the manufacture of a medicament for preventing, treating or lessening a disease mediated by FXR in a patient.

### BACKGROUND ART

Patent applications WO 2018024224A1 and CN107686486A have disclosed nitrogen-containing tricyclic compounds which can be used as FXR activity modulators, preparation methods and uses thereof. Wherein, compound 7, *i.e.*, 2-((5-cyclopropyl-3-(2,6-dichlorophenyl)isoxazol-4-yl)methoxy)-10*H*-spiro[benzo[6,7]oxepino[3,2 -b]pyridine-11,1'-cyclopropane]-7-carboxylic acid (the compound having Formula (I)), has been specifically disclosed.

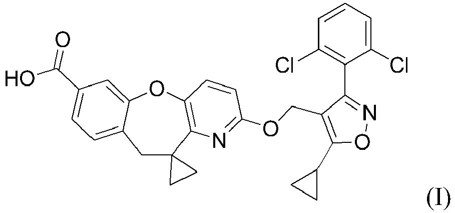

Patent applications WO2021104427A1, WO2021104421A1 and CN112876490A have disclosed the crystal forms of this compound.

It's known in the art that drug polymorphism is a common phenomenon in drug development, and is an important factor affecting the quality of drugs. Different crystal forms of the same drug may have significant differences in appearance, solubility, melting point, dissolution, bioavailability, etc., and may have different effects on the stability, bioavailability and efficacy of the drug. Therefore, in drug research and development, the crystal form of the drug should be fully investigated and studied.

### SUMMARY OF THE INVENTION

The present invention provides a new crystal form of 2-((5-cyclopropyl-3-(2,6-dichlorophenyl)isoxazol-4-yl)methoxy)-lOH spiro[benzo[6,7]oxepino[3,2 -b]pyridine-11,1'-cyclopropane]-7-carboxylic acid (the compound having Formula (I)), which is crystal form I of this compound. The crystal form has stable properties, no or almost no hygroscopicity, and good pharmacological properties (for example, good pharmacokinetic properties), and thus has excellent druggability. The present invention also provides a pharmaceutical composition comprising the crystal form I of the compound having Formula (I).

Specifically, the present invention relates to the crystal form I of the compound having Formula (I) and the pharmaceutical composition thereof, and the use of the crystal form I or the pharmaceutical composition in the manufacture of a medicament for preventing, treating or lessening a disease mediated by FXR in a patient. The crystal form I of the present invention can also be in the forms of solvates, such as hydrates.

In one aspect, provided herein is a crystal form of a compound having Formula (I), wherein the crystal form is crystal form I,

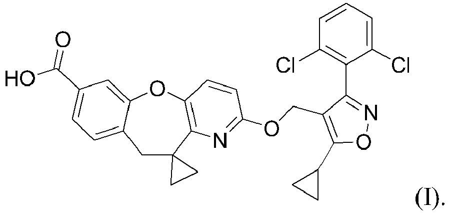

In some embodiments, the crystal form I exhibits the following characteristic X-ray powder diffraction peaks expressed as 2θ at 4.43°± 0.2°, 11.59°± 0.2°, 19.54°± 0.2°, 21.65°± 0.2°, 24.25°± 0.2°, 25.59°± 0.2°.

In some embodiments, the crystal form I exhibits the following characteristic X-ray powder diffraction peaks expressed as 2θ at 4.43°± 0.2°, 11.59°± 0.2°, 13.56°± 0.2°, 19.54°± 0.2°, 19.86°± 0.2°, 20.89°± 0.2°, 21.65°± 0.2°, 23.39°± 0.2°, 24.25°± 0.2°, 25.59°± 0.2°, 28.13°± 0.2°.

In some embodiments, the crystal form I exhibits the following characteristic X-ray powder diffraction peaks expressed as 2θ at 4.43°± 0.2°, 8.05°± 0.2°, 8.83°± 0.2°, 9.42°± 0.2°, 11.59°± 0.2°, 12.96°± 0.2°, 13.56°± 0.2°, 14.06°± 0.2°, 14.70°± 0.2°, 15.59°± 0.2°, 17.06°± 0.2°, 17.32°± 0.2°, 17.82°± 0.2°, 18.52°± 0.2°, 18.93°± 0.2°, 19.54°± 0.2°, 19.86°± 0.2°, 20.30°± 0.2°, 20.89°± 0.2°, 21.65°± 0.2°, 22.18°± 0.2°, 22.60°± 0.2°, 23.39°± 0.2°, 24.25°± 0.2°, 24.79°± 0.2°, 25.59°± 0.2°, 25.99°± 0.2°, 26.69°± 0.2°, 27.26°± 0.2°, 28.13°± 0.2°, 28.56°± 0.2°, 29.77°± 0.2°, 30.41°± 0.2°, 30.75°± 0.2°, 31.23°± 0.2°, 33.08°± 0.2°. In other embodiments, the crystal form I also exhibits the following characteristic X-ray powder diffraction peaks expressed as 2θ at 34.36°± 0.2°, 34.99°± 0.2°, 35.98°± 0.2°, 37.40°± 0.2°, 38.40°± 0.2°, 39.03°± 0.2°, 39.51°± 0.2°, 40.55°± 0.2°.

In some embodiments, the crystal form I exhibits the following characteristic X-ray powder diffraction peaks expressed as 2θ at 4.43°± 0.2°, 8.05°± 0.2°, 8.83°± 0.2°, 9.42°± 0.2°, 11.59°± 0.2°, 12.96°± 0.2°, 13.56°± 0.2°, 14.06°± 0.2°, 14.70°± 0.2°, 15.59°± 0.2°, 17.06°± 0.2°, 17.32°± 0.2°, 17.82°± 0.2°, 18.52°± 0.2°, 18.93°± 0.2°, 19.54°± 0.2°, 19.86°± 0.2°, 20.30°± 0.2°, 20.89°± 0.2°, 21.65°± 0.2°, 22.18°± 0.2°, 22.60°± 0.2°, 23.39°± 0.2°, 24.25°± 0.2°, 24.79°± 0.2°, 25.59°± 0.2°, 25.99°± 0.2°, 26.69°± 0.2°, 27.26°± 0.2°, 28.13°± 0.2°, 28.56°± 0.2°, 29.77°± 0.2°, 30.41°± 0.2°, 30.75°± 0.2°, 31.23°± 0.2°, 33.08°± 0.2°, 34.36°± 0.2°, 34.99°± 0.2°, 35.98°± 0.2°, 37.40°± 0.2°, 38.40°± 0.2°, 39.03°± 0.2°, 39.51°± 0.2°, 40.55°± 0.2°, 45.10°± 0.2°, 48.23°± 0.2°, 49.97°± 0.2°, 52.65°± 0.2°, 54.69°± 0.2°.

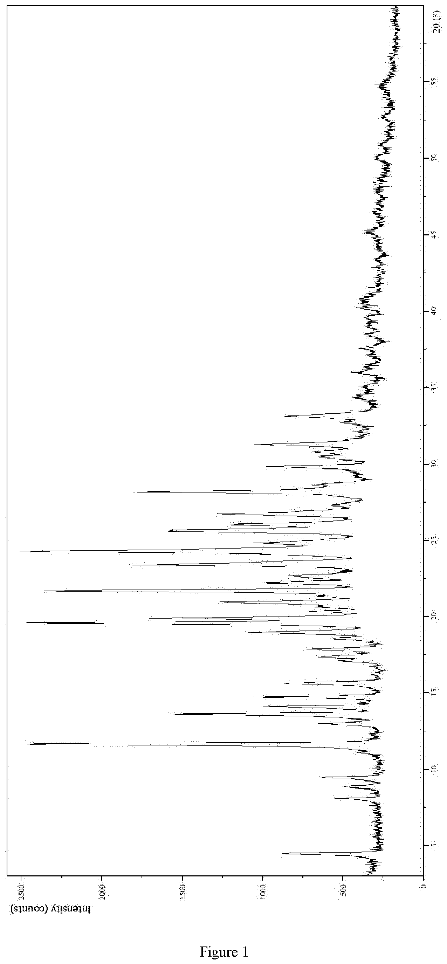

In some embodiments, the crystal form I has an X-ray powder diffraction pattern substantially as shown in FIG. 1.

In some embodiments, the crystal form I has a differential scanning calorimetry thermogram comprising endothermic peaks at 177.02 °C ± 3 °C and 195.73 °C ± 3 °C.

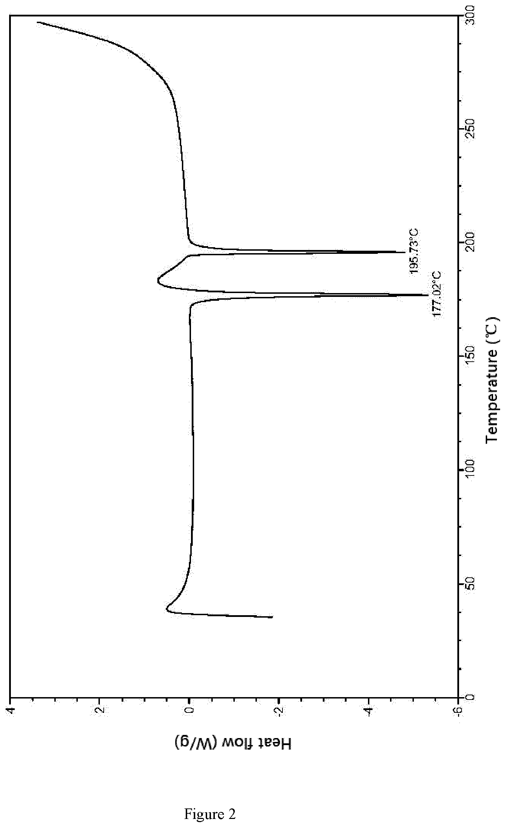

In some embodiments, the crystal form I has a differential scanning calorimetry thermogram substantially as shown in FIG. 2.

In some embodiments, when the crystal form I is heated to about 150 °C, the weight loss is about 0.321%, and there is an error tolerance of ± 0.1%.

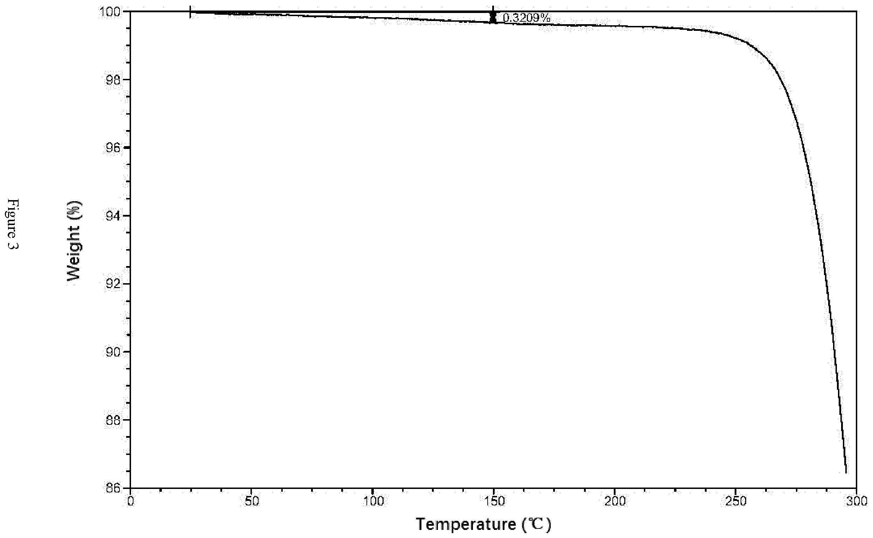

In some embodiments, the crystal form I has a thermogravimetric analysis curve substantially as shown in FIG. 3.

In one aspect, provided herein is a pharmaceutical composition comprising the crystal form of the present invention, and a pharmaceutically acceptable carrier, excipient, diluent, adjuvant or a combination thereof.

In another aspect, the present invention also relates to the use of the crystal form of the compound having Formula (I) or the pharmaceutical composition in manufacture of a medicament for preventing, treating or lessening a disease mediated by FXR in a patient; further, the use includes administering to the human or animal a therapeutically effective amount of the crystal form or the pharmaceutical composition of the present invention.

In some embodiments, the disease mediated by FXR is cardiovascular and cerebrovascular disease, a disease related to dyslipidemia, metabolic syndrome, hyperproliferative disease, fibrosis, inflammatory disease or a disease related to liver and gallbladder.

In other embodiments, the cardiovascular and cerebrovascular disease is atherosclerosis, acute myocardial infarction, venous occlusive disease, portal hypertension, pulmonary hypertension, heart failure, peripheral arterial occlusive disease, sexual dysfunction, stroke or thrombosis.

In other embodiments, the metabolic syndrome is insulin resistance, hyperglycemia, hyperinsulinemia, elevated levels of fatty acid or triglyceride in the blood, hyperlipidemia, obesity, hypertriglyceridemia, hypercholesterolemia, syndrome X, diabetic complications, atherosclerosis, hypertension, acute anemia, neutropenia, dyslipidemia, type II diabetes mellitus, diabetic nephropathy, diabetic neuropathy, diabetic retinopathy, dyslipidemia, or comorbidities of diabetes and abnormally high body mass index.

In other embodiments, the hyperproliferative disease is hepatocellular carcinoma, colonic adenocarcinoma, polyposis, colonic adenocarcinoma, breast cancer, membrane adenocarcinoma, Barrett's esophagus cancer, or other forms of gastrointestinal or liver neoplastic diseases.

In other embodiments, the fibrosis, inflammatory disease or disease related to liver and gallbladder is non-alcoholic fatty liver, non-alcoholic steatohepatitis, cholestasis, liver fibrosis, primary biliary cirrhosis, primary sclerosing cholangitis, progressive familial intrahepatic cholestasis, cystic fibrosis, drug-induced bile duct damage, gallstones, liver cirrhosis, hepatitis B, sebaceous gland disease, alcohol-induced liver cirrhosis, bile duct obstruction, gallstone disease, colitis, neonatal jaundice, nuclear jaundice, or overgrowth of intestinal bacteria.

In one aspect, the present invention relates to a method for preventing, treating or lessening a disease mediated by FXR in a patient, comprising administering to the patient a pharmaceutically acceptable effective amount of the crystal form or the pharmaceutical composition of the present invention.

In another aspect, the present invention relates to the crystal form of the compound having Formula (I) or the pharmaceutical composition for use in preventing, treating or lessening a disease mediated by FXR in a patient.

In another aspect, the present invention also relates to the preparation method of the crystal form of the compound having Formula (I).

The solvent used in crystal form production method of the present invention is not particularly restricted, and any solvent which dissolves the starting material to a degree and does not affect its properties is contained in the present invention. Additionally, many similar modifications in the art, equivalent replacements, or solvent, solvent composition and the solvent composition with different proportions which are equivalent to those described in the present invention, all are deemed to be included in the present invention. The present invention gives the preferred solvent for each reaction step.

The preparation of the crystal form of the present invention will be described in detail in the examples section. Meanwhile, the present invention provides property test experiments of the crystal form, such as pharmacokinetic experiments, stability experiments and hygroscopicity experiments, and the like. It can be seen from the experimental results that the crystal form I of the compound having Formula (I) of the present invention has good biological activity and high stability, and is suitable for pharmaceutical use. Specifically, the crystal form I of the present invention has more excellent pharmacokinetic properties, such as a higher exposure level; the crystal form I of the present invention is not susceptible to deliquescent due to high humidity, and it is convenient for the long-term storage of the medicine.

Compared with the prior arts, the crystal form of the present invention has better technical effects, such as better pharmacokinetic properties (such as higher exposure level and/or higher Cₘₐₓ value), and/or higher stability. For example, compared with the crystal forms disclosed in the prior art CN112876490A, the crystal form of the present invention has better effects in all aspects; the inventors found through research that among all the crystal forms disclosed in CN112876490A, the crystal form E is the most stable crystal form, however, its crystal structure changes after being placed at a high temperature of 60°C for 5 days, while the crystal form of the present invention is very stable under high temperature conditions and its crystal structure remains basically unchanged.

### DEFINITIONS AND GENERAL TERMINOLOGY

Unless otherwise indicated, all technical and scientific terms used in the present invention have the same meaning as commonly understood by one of ordinary skill in the art to which this invention pertains. All patents and publications referred to herein are incorporated by reference in their entirety. Although any methods and materials similar or identical to those described herein may be used in the practice or testing of the present invention, but the methods, apparatus and materials described in the present invention are preferred.

"Crystal form" or "crystalline form" refers to a solid having a highly regular chemical structure, including, but not limited to, mono- or multi-component crystals, and/or polymorphic compounds of compounds, solvates, hydrates, clathrates, eutectic, salt, salt solvent, salt hydrate. The crystalline form of the material can be obtained by a number of methods known in the field. Such methods include, but are not limited to, melt crystallization, melt cooling, solvent crystallization, crystallization in defined space, for example, in nanopores or capillaries, on a surface or template, for example, on a polymer, in the presence of additives such as co-crystallization counterions, removing solvent, dehydration, rapid evaporation, rapid cooling, slow cooling, vapor diffusion, sublimation, reaction crystallization, anti-solvent addition, grinding and solvent drop milling

"Solvent" refers to a substance (typically a liquid) that is capable of completely or partially dissolving another substance (typically a solid). Solvents for use in the practice of this invention include, but are not limited to, water, acetic acid, acetone, acetonitrile, benzene, chloroform, carbon tetrachloride, dichloromethane, dimethylsulfoxide, 1,4-dioxane, ethanol, ethyl acetate, butanol, *t*-butanol, *N*,*N*-dimethylacetamide, *N*,*N*-dimethylformamide, formamide, formic acid, heptane, hexane, isopropanol, methanol , methyl ethyl ketone, mesitylene, nitromethane, polyethylene glycol, propanol, pyridine, tetrahydrofuran, toluene, xylene, mixtures thereof, and the like.

"Anti-solvent" refers to a fluid that promotes the precipitation of a product (or product precursor) from a solvent. The anti-solvent may comprise a cold gas, or a fluid that promotes the precipitation of the fluid by chemical reaction or reduces the solubility of the product in the solvent; it may be the same liquid as the solvent but at a different temperature, or it may be a liquid different from the solvent.

"Solvate" refers to a compound that on a surface, in a lattice or having a solvent on a surface or in a lattice. The solvent can be water, acetic acid, acetone, acetonitrile, benzene, chloroform, carbon tetrachloride, dichloromethane, dimethylsulfoxide, 1,4-dioxane, ethanol, ethyl acetate, butanol, *t*-butanol, *N*,*N*-dimethylacetamide, *N*,*N*-dimethylformamide, formamide, formic acid, heptane, hexane, isopropanol, methanol, methyl ethyl ketone, methyl pyrrolidone, mesitylene, nitromethane, polyethylene glycol, propanol, pyridine, tetrahydrofuran, toluene, xylene, mixtures thereof, and the like. A specific example of the solvate is a hydrate in which the solvent on the surface, in the lattice or on the surface and in the lattice is water. On the surface, in the lattice or on the surface and in the lattice of the substance, the hydrate may or may not have any solvent other than water.

Crystal form can be identified by a variety of technical means, such as X-ray powder diffraction (XRPD), infrared absorption spectroscopy (IR), melting point method, differential scanning calorimetry (DSC), thermogravimetric analysis (TGA), Nuclear magnetic resonance, Raman spectroscopy, X-ray single crystal diffraction, dissolution calorimetry, scanning electron microscopy (SEM), quantitative analysis, solubility and dissolution rate.

X-ray powder diffraction (XRPD) can detect changes in crystal form, crystallinity, crystal state and other information, is a common means for identifying crystal form. In some embodiments, the crystalline form of the present invention is characterized by an XRPD map having certain peak positions, which is substantially as shown in the XRPD diagram provided in the drawings of the present invention at the same time, the 2θ of the XRPD pattern can be measured with an experimental error. The measurement of 2θ of the XRPD pattern may be slightly different between the different instruments and the different samples. Therefore, the value of 2θ cannot be regarded as absolute. According to the condition of the instrument used in this test, the diffraction peak has an error tolerance of ± 0.2°.

Differential Scanning Calorimetry (DSC) is a technique of measuring the energy difference between a sample and an inert reference (commonly used α-Al₂O₃) with temperature by continuously heating or cooling under program control. In some embodiments, the crystal form of the present invention is characterized by an DSC map having certain peak positions, which is substantially as shown in the DSC diagram provided in the drawings of the present invention. At the same time, the DSC pattern can be measured with an experimental error. The peak position and peak value of DSC pattern may be slightly different between the different instruments and the different samples. Therefore, the peak position or the peak value of the DSC endothermic peak can not be regarded as absolute. According to the condition of the instrument used in this test, the endothermic peak has an error tolerance of ±3°.

Thermogravimetric analysis (TGA) is a technique for measuring the quality of a substance with temperature under the control of a program. It is suitable for examining the process of the solvent loss or the samples sublimation and decomposition. It can be presumed that the crystal contains crystal water or crystallization solvent. The quality variety of the TGA curve shown depend on a number of factors, contains the sample preparation and the instrument. The quality variation of the TGA test may be slightly different between the different instruments and between the different samples. According to the condition of the instrument used in this test, there is a ± 0.1% error tolerance for the mass change.

In the context of the present invention, the 2θ values in the X-ray powder diffraction pattern are in degrees (°).

The term "substantially as shown in FIG." refers to at least 50%, or at least 60%, or at least 70%, or at least 80%, or at least 90%, or at least 95%, or at least 96%, or at least 97%, or at least 98%, or at least 99% of the peaks are shown in the X-ray powder diffraction pattern or DSC pattern.

The "peak" refers to a feature that a person skilled in the art can recognize without belonging to background noise when referring to a spectrum or/and data that appears in the figure.

The present invention relates to a new crystal form of 2-((5 -cyclopropyl-3 -(2,6-dichlorophenyl)isoxazol-4-yl)methoxy)-10*H*-spiro [benzo [6,7] oxepino [3,2 -*b*]pyridine-11,1'-cyclopropane]-7-carboxylic acid (the compound having Formula (I)), which exists in a substantially pure crystal form.

"Substantially pure" means that a crystalline form is substantially free of another or more crystalline forms, that means the purity of the crystalline form is at least 80%, or at least 85%, or at least 90%, or at least 93%, or at least 95%, or at least 96%, or at least 97%, or at least 98%, or at least 99%, or at least 99.5%, or at least 99.6%, or at least 99.7%, or at least 99.8%, or at least 99.9%, or crystal form containing other crystal form. The percentage of the other crystal form in the total volume or total weight of the crystal form is less than 20%, or less than 10%, or less than 5%, or less than 4%, or less than 3%, or less than 2%, or less than 1%, or less than 0.5%, or less than 0.1%, or less than 0.01%.

"Substantially free" means that the percentage of one or more other crystalline forms in the total volume or total weight of the crystalline form is less than 20%, or less than 10%, or less than 5%, or less than 4%, or less than 3%, or less than 2%, or less than 1%, or less than 0.5%, or less than 0. 1%, or less than 0.01%.

"Relative strength" or "relative peak height" in XRPD pattern means the ratio of the intensity of the other peaks to the intensity of the first strong peak when the intensity of the first strong peak in all the diffraction peaks of the X-ray powder diffraction pattern (XRD) is 100%.

In the context of the present invention, when used or whether or not used the word, such as "about", it means that within a given value or range of 10% or less, appropriately within 5%, especially within 1%. Or, for those of ordinary skill in the art, the term "about" or "approximately" means within an acceptable standard error range of the mean value. When a number with an N value is made public, any number within N +/- 1%, N +/- 2%, N +/- 3%, N +/- 5%, N +/- 7%, N +/-8%, or N +/- 10% will be opened clearly, wherein "+/-" means plus or minus.

In the present invention, "room temperature" refers to the temperature from about 10 °C to about 40 °C. In some embodiments, "room temperature" refers to a temperature from about 20 °C to about 30 °C; in other embodiments, "room temperature" refers to 20 °C, 22.5 °C, 25 °C or 27.5 °C, etc.

### PHARMACEUTICAL COMPOSITIONS, FORMULATIONS, ADMINISTRATION AND USES OF THE CRYSTAL FORM OF THE PRESENT INVENTION

The pharmaceutical composition of the present invention is characterized by comprising the crystal form of the compound having Formula (I), or a pharmaceutically acceptable carrier, adjuvant, or excipient. The amount of the crystal form of the compound in the pharmaceutical composition of the present invention can be effectively and detectably for treating or lessening the disease mediated by FXR in a patient.

As described above, the pharmaceutical composition disclosed herein further comprises a pharmaceutically acceptable carrier, an adjuvant, or an excipient, which, as used herein, includes any and all solvents, diluents, or other liquid vehicles, dispersion or suspension aids, surface active agents, isotonic agents, thickening or emulsifying agents, preservatives, solid binders, lubricants, and the like, as suited to the particular dosage form desired. As described in the following: In Remington: The Science and Practice of Pharmacy, 21st edition, 2005, ed. D.B. Troy, Lippincott Williams& Wilkins, Philadelphia, and Encyclopedia of Pharmaceutical Technology, eds. J. Swarbrick and J. C. Boylan, 1988-1999, Marcel Dekker, New York, both of which are herein incorporated by reference in their entireties, discloses various carriers used in formulating pharmaceutically acceptable compositions and known techniques for the preparation thereof. Except insofar as any conventional carrier medium incompatible with the compound or the crystal form disclosed herein, such as by producing any undesirable biological effect or otherwise interacting in a deleterious manner with any other components of the pharmaceutically acceptable composition, its use is contemplated to be within the scope of this invention.

The crystal form of the present invention can be used as an active ingredient and uniformly combined in a mixture with a drug carrier according to conventional drug compounding technology. The carrier can be in various forms according to the formulation required for administration, such as oral or parenteral (including intravenous). When preparing a composition for oral dosage form, any conventional pharmaceutical medium can be used, for example, water, glycol, oil, alcohol, fragrance, preservative, colorant, etc., can be used when preparing oral liquid medicaments such as suspensions, elixirs and solutions; or, for example, starch, sugar, microcrystalline cellulose, diluents, granulating agents, lubricants, binders, disintegrants, etc., can be used in the preparation of oral solid preparations such as powders, hard capsules, soft capsules and tablets, among which solid oral preparations are more preferable than liquid pharmaceuticals.

Because tablets and capsules are easy to take, they represent the most advantageous oral dosage unit form, in this case solid pharmaceutical carriers are obviously used. If necessary, tablets can be coated with standard aqueous or non-aqueous techniques.

A variety of other materials can be present as coatings or to modify the shape of the dosage unit. For example, tablets can be coated with shellac, sugar, or both. In addition to the active ingredients, syrups or elixirs may contain sucrose as a sweetener, methyl or propyl 4-hydroxybenzoate as preservatives, dyes and flavoring agents (for example, cherry flavored or orange flavored).

The crystal form of the present invention can also be administered parenterally. A solution or suspension of these active substances can be prepared by mixing appropriately with a surfactant (such as hydroxypropyl cellulose) in water. In glycerin, liquid polyethylene glycol and mixtures thereof, and in oil, dispersants can also be prepared. Under normal conditions of storage and use, these preparations contain a preservative to prevent the growth of microorganisms.

Pharmaceutical forms suitable for injection include sterile aqueous solutions or dispersions and sterile powders for the immediate preparation of sterile injectable solutions or dispersions. In all cases, the pharmaceutical form must be sterile and must be a fluid in an easily injectable form. It must be stable under the conditions of manufacture and storage and must be preserved under conditions that resist the contaminating action of microorganisms such as bacteria and fungi. The carrier can be a solvent or dispersion medium containing, for example, water, ethanol, polyol (e.g. glycerol, propylene glycol and liquid polyethylene glycol), suitable mixtures thereof, and vegetable oils.

Any suitable method of administration can be used to provide an effective dose of the crystal form of the present invention to mammals, especially humans. For example, oral, rectal, topical, parenteral, intraocular, pulmonary, and nasal administration methods can be used. Dosage forms include tablets, lozenges, dispersions, suspensions, solutions, capsules, emulsions, ointments, aerosols, and the like.

The crystal form of the present invention or pharmaceutical composition thereof can be effectively used for preventing, managing, treating, or lessening the disease mediated by FXR in patients, especially effectively used for treating non-alcoholic fatty liver (NAFLD) and non-alcoholic steatohepatitis (NASH), obesity, hypertriglyceridemia, atherosclerosis, chronic intrahepatic cholestasis, primary biliary cirrhosis (PBC), primary sclerosing cholangitis (PSC), progressive Familial cholestasis (PFIC), drug-induced bile duct damage, gallstones, liver cirrhosis, hepatitis B, sebaceous gland disease, alcohol-induced liver cirrhosis, cystic fibrosis, bile duct obstruction, gallstone disease, liver fibrosis, dyslipidemia, atherosclerosis, type II diabetes mellitus, diabetic nephropathy, diabetic neuropathy, diabetic retinopathy, peripheral arterial obstructive disease (PAOD), colitis, neonatal Jaundice, nuclear jaundice, venous occlusive disease, portal hypertension, metabolic syndrome, acute myocardial infarction, acute stroke, thrombosis, hypercholesterolemia, intestinal bacterial overgrowth, erectile dysfunction, gastrointestinal tumor and liver tumor, etc.

### DESCRIPTION OF THE DRAWINGS

FIG. 1 is an X-ray powder diffraction (XRPD) pattern of the crystal form I of the compound having Formula (I) prepared according to the method of Example 1 of the present invention.
FIG. 2 is a differential scanning calorimetry (DSC) thermogram of the crystal form I of the compound having Formula (I) prepared according to the method of Example 1 of the present invention.
FIG. 3 is a thermogravimetric analysis (TGA) thermogram of the crystal form I of the compound having Formula (I) prepared according to the method of Example 1 of the present invention.
FIG. 4 is a Dynamic Vapor Sorption (DVS) graph of the crystal form I of the compound having Formula (I) prepared according to the method of Example 1 of the present invention.

### EXAMPLES

The present invention will now be further described by way of example without limiting the present invention to the scope of the present invention.

The X-ray powder diffraction analysis method used in the present invention was an Empyrean diffractometer, and an X-ray powder diffraction pattern was obtained using Cu-Kα radiation (45 KV, 40 mA). The powdery sample was prepared as a thin layer on a monocrystalline silicon sample rack and placed on a rotating sample stage, analyzed at a rate of 0.0167° steps in the range of 3 °-60 °. Data Collector software was used to collect data, High Score Plus software was used to process data, Data Viewer software was used to read data.

The differential scanning calorimetry (DSC) analysis method used in the present invention is a differential scanning calorimeter using a TA Q2000 module with a thermal analysis controller. Data were collected and analyzed using TA Instruments Thermal Solutions software. Approximately 1-5 mg of the sample was accurately weighed into a specially crafted aluminum crucible with a lid and analyzed from room temperature to about 300 ° C using a linear heating device at 10 ° C/min. During use, the DSC chamber was purged with dry nitrogen.

The thermogravimetric analysis (TGA) analysis method used in the present invention is: performing a thermogravimetric analysis using a TA Q500 module with a thermal analysis controller. Data were collected and analyzed using TA Instruments Thermal Solutions software. Approximately 10-30 mg of the sample was placed into a platinum crucible and analyzed from room temperature to about 300 °C using a linear heating device at 10 °C/min. During use, the DSC chamber was purged with dry nitrogen.

The hygroscopicity of the present invention is measured using the DVS INT-Std dynamic moisture and gas adsorption analyzer of the Surface Measurement Systems Company in UK. The humidity test range was 0%-95%, the air flow was 200 mL/min, the temperature was 25 °C, test point: take one test point per liter of 5% humidity.

### EXAMPLES

Original sample: the compound having Formula (I) (2-((5-cyclopropyl-3-(2,6-dichlorophenyl)isoxazol-4-yl)methoxy)-10/7-spiro[benzo[6,7]oxepino[3, 2-b]pyridine-11,1'-cyclopropane]-7-carboxylic acid) was prepared by referring to the method of Example 9 in the patent application CN107686486A.

### Example

### Example 1 Crystal form I of the compound having Formula (I)

### 1. Preparation of crystal form I

The original sample (566.4 mg) was suspended in chloroform (5.7 mL), the mixture was stirred at room temperature to dissolve, and then cooled to -10°C. To the mixture was added n-heptane (8.0 mL), the resulting mixture was stirred for 4 hrs and filtered with suction, the filter cake was washed with n-heptane (4.0 mL×2), pumped until almost dry, and dried under vacuum at room temperature overnight to obtain a white solid (468.2 mg, yield 83.6%).

### 2. Identification of crystal form I

(1) Analysis and identification by Empyrean X-ray powder diffraction (XRPD) using Cu-Kα radiation with the following characteristic peaks at an angle of 2θ: 4.43°, 8.05°, 8.83°, 9.42°, 11.59°, 12.96°, 13.56°, 14.06°, 14.70°, 15.59°, 17.06°, 17.32°, 17.82°, 18.52°, 18.93°, 19.54°, 19.86°, 20.30°, 20.89°, 21.65°, 22.18°, 22.60°, 23.39°, 24.25°, 24.79°, 25.59°, 25.99°, 26.69°, 27.26°, 28.13°, 28.56°, 29.77°, 30.41°, 30.75°, 31.23°, 33.08°, 34.36°, 34.99°, 35.98°, 37.40°, 38.40°, 39.03°, 39.51°, 40.55°, 45.10°, 48.23°, 49.97°, 52.65° and 54.69°. There is an error tolerance of ± 0.2 °. The X-ray powder diffraction of the crystal form I prepared by the method of the example was substantially as shown in FIG. 1.

(2) Analysis and identification by TA Q2000 Differential Scanning Calorimetry: the scanning speed is 10 ° C/min and contains the endothermic peaks of 177.02 °C and 195.73 °C. There is an error tolerance of ± 3 ° C. The differential scanning calorimetry thermogram of the crystal form I prepared by the method of the example was substantially as shown in FIG. 2.

(3) The crystal form I was analyzed and identified by Thermogravimetric Analysis (TGA) using TA Q500: the heating rate was 10°C/min, the weight loss was 0.321%, and there was an error tolerance of ±0.1%. The thermogravimetric Analysis graph of the crystal form I prepared by the method of the example was substantially as shown in FIG. 3.

### Example 2 The pharmacokinetic experiment of the crystal form of the present invention

The crystal form of the compound having Formula (I) was filled into capsules, which were used for oral administration.

Three male Beagle dogs weighing 8-12 kg were orally administered with capsules containing the test sample at a dose of 5 mg/kg, and blood samples were taken at time points of 0.25 h, 0.5 h, 1.0 h, 2.0 h, 4.0 h, 6.0 h, 8.0 h and 24 h. Standard curve was plotted based on concentrations of the samples in a suitable range, the concentration of the test sample in the plasma sample was measured and quantified by AB SCIEX API4000 LC-MS/MS at MRM mode. Pharmacokinetic parameters were calculated according to drug concentration -time curve using a noncompartmental method by WinNonLin 6.3 software. Results were shown in table 1.

**Table 1 Pharmacokinetic data of the crystal form I of the present invention**

| Test sample | AUCₗₐₛₜ (h*ng/ml) | Cₘₐₓ (ng/ml) | Tₘₐₓ (h) |
|---|---|---|---|
| Crystal form I | 1020 | 389 | 1.67 |

### Experiment Conclusion:

It can be seen from Table 1 that the crystal form I of the present invention has a relatively large exposure in Beagle dogs and has good pharmacokinetic properties.

### Example 3 The stability experiment of the crystal form of the present invention

(1) High-temperature test: A batch of the test sample was taken into a flat weighing bottle, divided into ≤ 5 mm thick thin layer. The sample was placed at 60°C for 30 days. The sample was then sampled at day 5, day 13 and day 30, and the change of the sample's color was observed. The purity of the sample was detected by HPLC.
(2) High-humidity test: take a batch of the test sample into the flat weighing bottle, divided into ≤ 5 mm thick thin layer. The samples were sampled at 25 ° C and RH for 90% ± 5% for 30 days. The samples were sampled at day 5, 13 and day 30, and the sample color was observed. The purity of the sample was determined by HPLC.
(3) Photostability test: A batch of the test sample was taken into a flat weighing bottle, divided into ≤ 5 mm thick thin layer. The sample was placed in an opened light box (with UV) at the illuminance 4500±500lx, UV light ≥0.7w/m² for 30 days. The sample was then sampled at day 5, day 13 and day 30, and the change of the sample's color was observed. The purity of the sample was detected by HPLC.

The changes of appearance and chemical purity of the crystal form I in the stability experiment were shown in Table 2.

**Table 2 The stability experiment of the crystal form I of the present invention**

| Condition | | High-temperature | | | High-humidity | | | Lighting | | |
|---|---|---|---|---|---|---|---|---|---|---|
| Project | day 0 | day 5 | day 13 | day 30 | day 5 | day 13 | day 30 | day 5 | day 13 | day 30 |
| Appearance | White powder | White powder | White powder | White powder | White powder | White powder | White powder | White powder | White powder | White powder |
| Purity (%) | 99.88 | 99.83 | 99.91 | 99.70 | 99.81 | 99.70 | 99.82 | 99.88 | 99.59 | 99.83 |

### Experiment Conclusion:

Under the conditions of high-temperature, high-humidity and/or lighting, the crystal form I of the present invention has no obvious changes in the appearance and chemical purity, thus has good stability effect, and it is suitable for pharmaceutical use. In addition, the structure of the crystal form I of the present invention is also very stable, and the crystal structure remains basically unchanged under high temperature, high humidity and/or lighting conditions.

### Example 4 The hygroscopicity experiment of the crystal form of the present invention

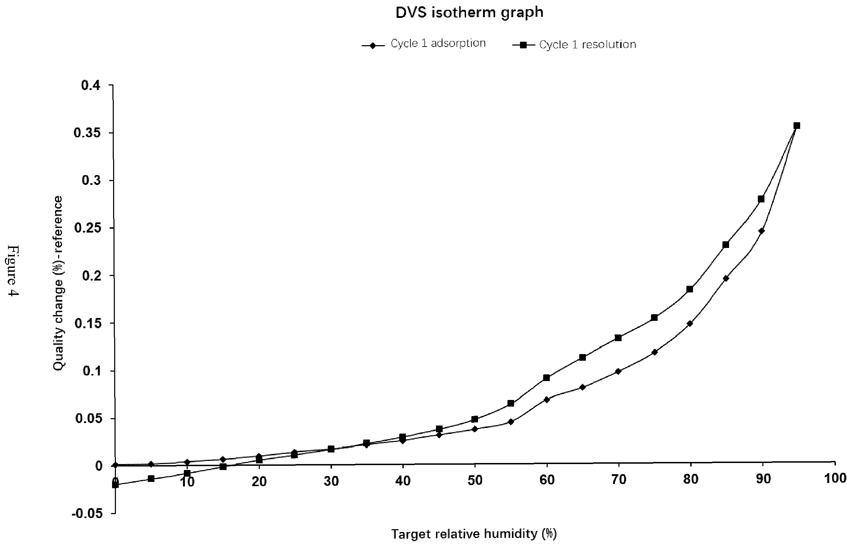

An appropriate amount of the test sample was taken and the hygroscopicity of the test sample was tested by dynamic moisture adsorption device. The experimental results are shown in Figure 4. The description of the hygroscopic characteristics and the definition of the hygroscopic weight gain (Appendix 9103 of Chinese Pharmacopoeia 2020 Edition, Guidelines for Drug Hygroscopicity Tests, experimental conditions: 25 °C ± 1 °C, 80% ± 2% relative humidity) are described in the table below:

### Description of hygroscopic characteristics and definition of hygroscopic weight gain

| Hygroscopic characteristics | Hygroscopic weight gain |
|---|---|
| Deliquescence | Absorb enough water to form a liquid |
| Highly hygroscopicity | Not less than 15% |
| Hygroscopicity | Less than 15% but not less than 0.2% |
| Lightly hygroscopicity | Less than 2% but not less than 0.2% |
| No or almost no hygroscopicity | Less than 0.2% |

The experimental results show that the weight gain of the crystal form I of the present invention is less than 0.2% after equilibrium under the condition of a relative humidity of 80%. According to the criterion of hygroscopic weight gain, it has no or almost no hygroscopicity. Therefore, the crystal form I of the present invention is not susceptible to deliquescent under the influence of high humidity.

The foregoing description is merely a basic illustration of the present invention and any equivalent transformation made in accordance with the technical solution of the present invention is intended to be within the scope of the present invention.

Reference throughout this specification to "an embodiment," "some embodiments," "one embodiment", "another example," "an example," "a specific example," or "some examples," means that a particular feature, structure, material, or characteristic described in connection with the embodiment or example is included in at least one embodiment or example of the present disclosure. In this specification, the schematic expressions of the above terms are not necessarily directed to the same embodiment or example. Furthermore, the particular features, structures, materials, or characteristics may be combined in any suitable manner in one or more embodiments or examples. In addition, those skilled in the art can integrate and combine different embodiments, examples or the features of them as long as they are not contradictory to one another.

Although explanatory embodiments have been shown and described, it would be appreciated by those skilled in the art that the above embodiments cannot be construed to limit the present disclosure, and changes, alternatives, and modifications can be made in the embodiments without departing from spirit, principles and scope of the present disclosure.

## Claims

1. A crystal form of a compound having Formula (I), which is crystal form I, 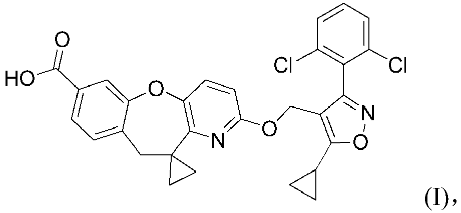 wherein the crystal form I exhibits the following characteristic X-ray powder diffraction peaks expressed as 2θ at 4.43°± 0.2°, 11.59°± 0.2°, 19.54°± 0.2°, 21.65°± 0.2°, 24.25°± 0.2°, 25.59°± 0.2°.

2. The crystal form according to claim 1, wherein the crystal form I exhibits the following characteristic X-ray powder diffraction peaks expressed as 2θ at 4.43°± 0.2°, 11.59°± 0.2°, 13.56°± 0.2°, 19.54°± 0.2°, 19.86°± 0.2°, 20.89°± 0.2°, 21.65°± 0.2°, 23.39°± 0.2°, 24.25°± 0.2°, 25.59°± 0.2°, 28.13°± 0.2°.

3. The crystal form according to claim 1 or 2, wherein the crystal form I exhibits the following characteristic X-ray powder diffraction peaks expressed as 2θ at 4.43°± 0.2°, 8.05°± 0.2°, 8.83°± 0.2°, 9.42°± 0.2°, 11.59°± 0.2°, 12.96°± 0.2°, 13.56°± 0.2°, 14.06°± 0.2°, 14.70°± 0.2°, 15.59°± 0.2°, 17.06°± 0.2°, 17.32°± 0.2°, 17.82°± 0.2°, 18.52°± 0.2°, 18.93°± 0.2°, 19.54°± 0.2°, 19.86°± 0.2°, 20.30°± 0.2°, 20.89°± 0.2°, 21.65°± 0.2°, 22.18°± 0.2°, 22.60°± 0.2°, 23.39°± 0.2°, 24.25°± 0.2°, 24.79°± 0.2°, 25.59°± 0.2°, 25.99°± 0.2°, 26.69°± 0.2°, 27.26°± 0.2°, 28.13°± 0.2°, 28.56°± 0.2°, 29.77°± 0.2°, 30.41°± 0.2°, 30.75°± 0.2°, 31.23°± 0.2°, 33.08°± 0.2°.

4. The crystal form according to any one of claims 1 to 3, wherein the crystal form I has an X-ray powder diffraction pattern substantially as shown in FIG. 1.

5. The crystal form according to any one of claims 1 to 4, wherein the crystal form I has a differential scanning calorimetry thermogram comprising endothermic peaks at 177.02 °C± 3 °C and 195.73 °C ± 3 °C.

6. The crystal form according to any one of claims 1 to 5, wherein the crystal form I has a differential scanning calorimetry thermogram substantially as shown in FIG. 2.

7. A pharmaceutical composition comprising the crystal form of any one of claims 1 to 6, and a pharmaceutically acceptable carrier, excipient, diluent, adjuvant or a combination thereof.

8. Use of the crystal form of any one of claims 1 to 6 or the pharmaceutical composition of claim 7 in the manufacture of a medicament for preventing, treating or lessening a disease mediated by FXR in a patient.

9. The use according to claim 8, wherein the disease mediated by FXR is cardiovascular and cerebrovascular disease, a disease related to dyslipidemia, metabolic syndrome, hyperproliferative disease, fibrosis, inflammatory disease or a disease related to liver and gallbladder.

10. The use according to claim 9, wherein the cardiovascular and cerebrovascular disease is atherosclerosis, acute myocardial infarction, venous occlusive disease, portal hypertension, pulmonary hypertension, heart failure, peripheral arterial occlusive disease, sexual dysfunction, stroke or thrombosis;
wherein the metabolic syndrome is insulin resistance, hyperglycemia, hyperinsulinemia, elevated levels of fatty acid or glycerol in the blood, hyperlipidemia, obesity, hypertriglyceridemia, hypercholesterolemia, syndrome X, diabetic complications, atherosclerosis, hypertension, acute anemia, neutropenia, dyslipidemia, type II diabetes mellitus, diabetic nephropathy, diabetic neuropathy, diabetic retinopathy, dyslipidemia, or comorbidities of diabetes and abnormally high body mass index;
wherein the hyperproliferative disease is hepatocellular carcinoma, colonic adenocarcinoma, polyposis, colonic adenocarcinoma, breast cancer, membrane adenocarcinoma, Barrett's esophagus cancer, or other forms of gastrointestinal or liver neoplastic diseases;
wherein the fibrosis, inflammatory disease or disease related to liver and gallbladder is non-alcoholic fatty liver, non-alcoholic steatohepatitis, cholestasis, liver fibrosis, primary biliary cirrhosis, primary sclerosing cholangitis, progressive familial intrahepatic cholestasis, cystic fibrosis, drug-induced bile duct damage, gallstones, liver cirrhosis, hepatitis B, sebaceous gland disease, alcohol-induced liver cirrhosis, bile duct obstruction, gallstone disease, colitis, neonatal Jaundice, nuclear jaundice, or overgrowth of intestinal bacteria.
